# EUROPEAN PATENT APPLICATION

(11) **EP 3 791 830 A1**
(43) Date of publication of application: **17.03.2021**
(21) Application number: 20196410.3
(22) Date of filing: 16.09.2020
(51) Int. Cl.: A61F 2/42, A61F 2/30

(54) **ANKLE PROSTHESIS WITH ANATOMIC RANGE OF MOTION**

(30) Priority: 16.09.2019 US 201962901068 P
(71) Applicant: Kinos Medical Inc., Wayne, PA 19087 (US)
(72) Inventor: GARVEY, Brian, Bryn Mawr, PA 19010 (US); PADMANABHAN, Deepak, Philadelphia, PA 19104 (US)
(74) Representative: Rummler, Felix

(57) **Abstract**

An ankle prosthesis implant (1) is disclosed herein. The ankle prosthesis implant includes a talar implant (100) defining a superior bearing surface (102). The superior bearing surface includes a convex portion (104) and a concave portion (106). The convex portion is defined in an anterior-posterior direction when viewed from a sagittal plane and has a neutral axis (X1) defined in a coronal plane at an anterior-posterior midline of the talar implant and extending in a medial-lateral direction. The concave portion is defined in the medial-lateral direction when viewed from the coronal plane, and the concave portion is swept about a secondary axis (X2) that is angled relative to the neutral axis (X1) upwards towards a medial end (106b) of the talar implant by an angle (θ).

## Description

### INCORPORATION BY REFERENCE

The following document is incorporated by reference as if fully set forth herein: US Provisional Patent Application 62/901,068, filed September 16, 2019.

### FIELD OF INVENTION

The present invention relates to an ankle prosthesis implant, and is more specifically directed to a talar implant, mating bearing component, and tibial implant.

### BACKGROUND

Ankle prosthetic implants are well known. Some known existing implants are disclosed by US Patents 8,715,362 and 9,925,054; and US Pub. 2014/0107799. One specific design for ankle prosthetic implants includes a talar implant component that defines a saddle shaped bearing surface.

Known talar implants suffer from limitations regarding articulation. In particular, there is a need for a talar implant that provides improved flexion and extension of the ankle joint, as well as the requisite internal and external rotation. Existing implants only allow for limited flexion and extension (*i.e.* hinging motion).

It would be desirable to provide an ankle prosthetic device that both allows a patient to move their repaired ankle within the desired range of motion, and specifically provides a wide range of axial rotation and planar rotation.

### SUMMARY

An ankle prosthesis implant is disclosed herein. The ankle prosthesis implant includes a talar implant defining a superior bearing surface. The superior bearing surface includes a convex portion or curvature and a concave portion or curvature. The convex portion is defined in an anterior-posterior direction when viewed from a sagittal plane and has a neutral axis (X1) defined in a coronal plane approximately at the anterior-posterior midline of the talar implant and extending in a medial-lateral direction. The term approximately, as used in this context, means in the middle 50% (+/- 5%) of the anterior-posterior length of the talar implant. The concave portion is defined in the medial-lateral direction when viewed from the coronal plane, and the concave portion is swept about a secondary axis (X2) that is angled relative to the neutral axis (X1) upwards in the medial-lateral direction towards a medial end of the talar implant by an angle (θ).

In one embodiment, the angle (θ) of the secondary axis (X2) relative to the neutral axis (X1) is between 1° to 30°. In another embodiment, the angle (θ) of the secondary axis (X2) relative to the neutral axis (X1) is tilted upwards by 5° to 10° toward the medial end of the talar implant. The angle (θ) of the secondary axis (X2) relative to the neutral axis (X1) can also be tilted upwards 7° toward the medial end of the talar implant. In another embodiment, the angle (θ) of the secondary axis (X2) relative to the neutral axis (X1) is tilted upwards by at least 5° toward the medial end of the talar implant.

In one embodiment, the concave portion has a single radius of curvature when viewed from the coronal plane. In other embodiments, the concave portion has multiple radii of curvature when viewed from the coronal plane.

The geometry of the talar implant is selected to provide maximum bone coverage and appropriate range of motion. The width (W_{S}) of the concave portion in the medial-lateral direction when viewed from an axial plane is preferably less than an overall width (W_{O}) of the talar implant in the medial-lateral direction when viewed from the axial plane.

Siderails can be provided at a lateral end and a medial end of the concave portion, and the siderails each partially define an outermost medial edge and an outermost lateral edge of the talar implant. The siderails are angled by a siderail angle (β) from a vertical plane (P) extending in a superior-inferior direction when viewed from the coronal plane. In one embodiment, the siderail angle (β) is between -30° to 60°. The siderails preferably each have a siderail height (H_{SR}) in a superior-inferior direction when viewed in the coronal plane that is at least 0.5 mm. In another embodiment, the siderail height (H_{SR}) is at least 1%-15% of a total height (H_{T}) of the talar implant in the superior-anterior direction when viewed in the coronal plane.

The contour of the convex portion can include varying degrees of curvature. In one embodiment, the convex portion has a single radius of curvature when viewed from the sagittal plane. In another embodiment, the convex portion has multiple radii of curvature. In another embodiment, the convex portion has a region at its anterior end where the convex curvature transitions to a concave curvature

The talar implant defines an inferior bone contacting region that includes at least one bone attachment protrusion. The at least one bone attachment protrusion is dimensioned to extend inside of a bone.

The ankle prosthesis implant also includes a bearing component defining a mating surface that abuts the superior bearing surface and articulates with the talar implant. The mating surface of the bearing component includes a concave bearing surface when viewed in the sagittal plane and a convex bearing surface when viewed in the coronal plane. In one embodiment the bearing surface has a width in the medial-lateral direction that is less than the width of the talar component in the medial-lateral direction.

The ankle prosthesis implant also includes a tibial implant. The tibial implant includes at least one dorsal fin that extends in the medial-lateral direction and extends perpendicular from a superior planar surface of the tibial implant. The at least one dorsal fin includes at least one of a void, opening, or hole, which promotes attachment with a patient's bone.

The ankle prosthesis implant disclosed herein generally provides axial rotation with flexion and extension of the ankle joint, as well as planar rotation, *i.e.* when the ankle is pointed downward.

The ankle prosthesis implant also allows for the overall axis of rotation to move, such that movement is not constrained to a single cylindrical plane.

Additional embodiments are disclosed herein.

### BRIEF DESCRIPTION OF THE DRAWINGS

The foregoing Summary and the following Detailed Description will be better understood when read in conjunction with the appended drawings, which illustrate a preferred embodiment of the invention. In the drawings:
Figure 1 is a perspective view of an embodiment of an ankle prosthesis implant.
Figure 2 is a cross-sectional view of the ankle prosthesis implant of Figure 1.

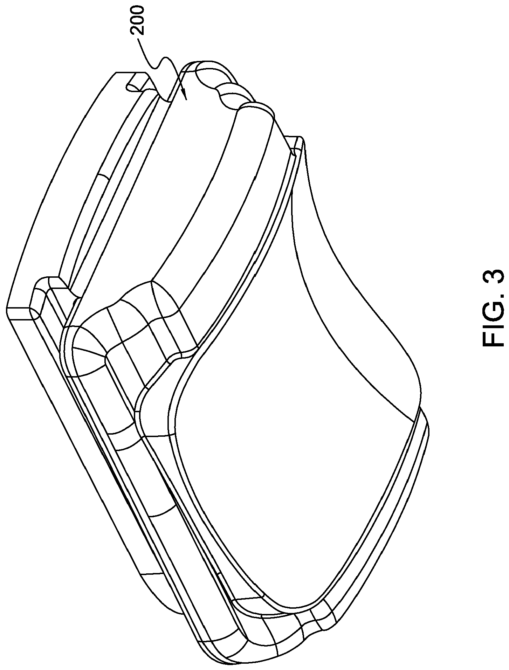

Figure 3 is a lower perspective view of a bearing component of the ankle prosthesis implant.

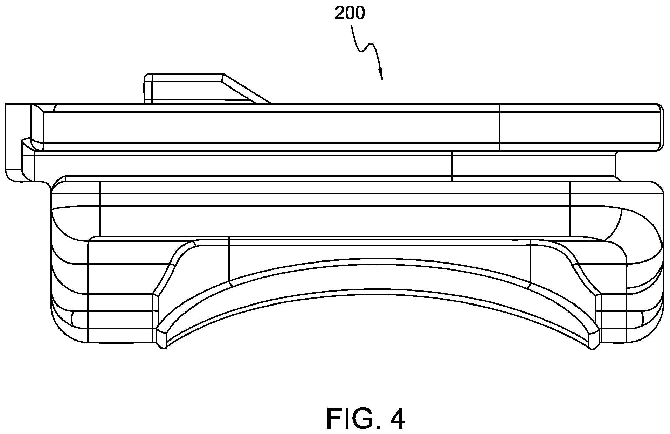

Figure 4 is a lateral or side view of the bearing component of Figure 3 when viewed in the sagittal plane.
Figure 5A is a frontal or anterior view of a talar implant viewed from the coronal plane.
Figure 5B is a top or superior view of the talar implant of Figure 5A as viewed from the axial plane.
Figure 5C is a perspective view of the talar implant of Figures 5A and 5B.
Figure 5D is a side or lateral view of the talar implant of Figures 5A-5C as viewed from the sagittal plane.
Figure 5E is another perspective view of the talar implant of Figures 5A-5D.
Figure 5F is another side or lateral view of the talar implant of Figures 5A-5E viewed from the sagittal plane through a cross-sectional line 5F-5F shown in Figure 5B.
Figure 5G is a side or lateral view of a talar implant having a modified convex portion.
Figure 6A is a side perspective view of the bearing component.
Figure 6B is a cross-sectional view in the coronal plane of the bearing component of Figure 6A.
Figure 6C is a cross sectional view in the sagittal plane of the bearing component of Figures 6A and 6B.
Figure 6D is an inferior or bottom view of the bearing component as viewed from the axial plane.
Figure 6E is another cross-sectional view in the coronal plane of the bearing component of Figures 6A-6D.
Figure 7A is a bottom perspective view of a tibial implant.
Figure 7B is another perspective view of the tibial implant of Figure 7A.
Figure 7C is a third perspective view of the tibial implant of Figures 7A and 7B.
Figure 7D is a lateral or side view of the tibial component of Figures 7A-7C when viewed in the sagittal plane.

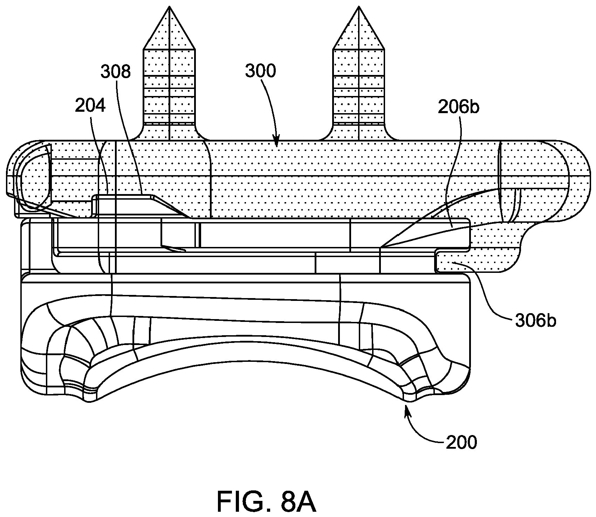

Figure 8A is a partial see-through lateral or side view of the tibial component and bearing component when viewed in the sagittal plane.

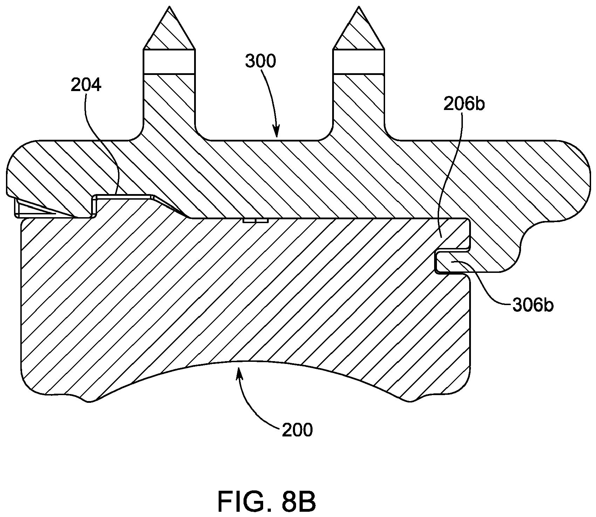

Figure 8B is a cross-sectional lateral or side view of the tibial component and bearing component when viewed in the sagittal plane.
Figure 9A is a simplified anterior view of the talar implant when viewed in the coronal plane.
Figure 9B is another anterior view of the talar implant when viewed in the coronal plane.
Figure 9C is a perspective view of the talar implant of Figures 9A and 9B.
Figure 9D is a schematic view of a profile defined by a concave portion of a bearing surface of the talar implant of Figures 9A-9C.
Figure 9E is a perspective view of the talar implant of Figures 9A-9D further illustrating the coronal, sagittal, and axial planes.
Figure 9F is a front or anterior view of the talar implant of Figures 9A-9E when viewed in the coronal plane.
Figure 9G is a top or superior view of the talar implant of Figures 9A-9F when viewed in the axial plane.
Figure 9H is a perspective view of the talar implant of Figures 9A-9G with the coronal plane annotated.
Figure 9I is another perspective view of the talar implant of Figures 9A-9H with the axial plane annotated.
Figure 9J is a third perspective view of the talar implant of Figures 9A-9I with the coronal plane, sagittal plane, and axial plane annotated.
Figure 9K is a front or anterior view of the talar implant of Figures 9A-9J.
Figure 9L is a perspective view of the talar implant of Figures 9A-9K.
Figure 10A is a perspective view of an embodiment of a talar implant according to another embodiment.
Figure 10B is another perspective view of the talar implant of Figure 10A.
Figure 10C top or superior view of the talar implant of Figures 10A and 10B as viewed from the axial plane.
Figure 10D is a side or lateral view of the talar implant of Figures 10A-10C as viewed from the sagittal plane
Figure 10E is a frontal or anterior view of a talar implant viewed from the coronal plane.
Figure 10F is another side or lateral view of the talar implant of Figures 10A-10E viewed from the sagittal plane through a cross-sectional plane "10F-10F" illustrated in Figure 10E.

### DETAILED DESCRIPTION OF THE PREFERRED EMBODIMENTS

Certain terminology is used in the following description for convenience only and is not limiting. The words "front," "upper" and "lower" designate directions in the drawings to which reference is made. A reference to a list of items that are cited as "at least one of a, b, or c" (where a, b, and c represent the items being listed) means any single one of the items a, b, or c, or combinations thereof.

The coronal, sagittal, and axial planes are illustrated throughout the drawings and referenced throughout this disclosure. These directional terms are used according to their generally accepted definitions as used in the medical field unless explicitly clarified herein. The terms superior/inferior, medial/lateral, and posterior/anterior are similarly used according to the generally accepted definitions as used in the medical field, unless explicitly clarified herein. The drawings include further clarifications regarding these directions and planes to the extent it is believed necessary. The terms top/bottom are sometimes used interchangeably with superior/inferior, and the term side is sometimes used interchangeably with medial/lateral.

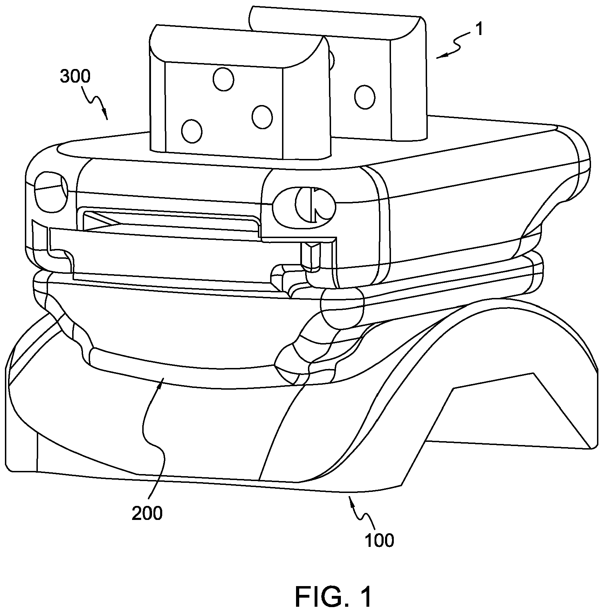

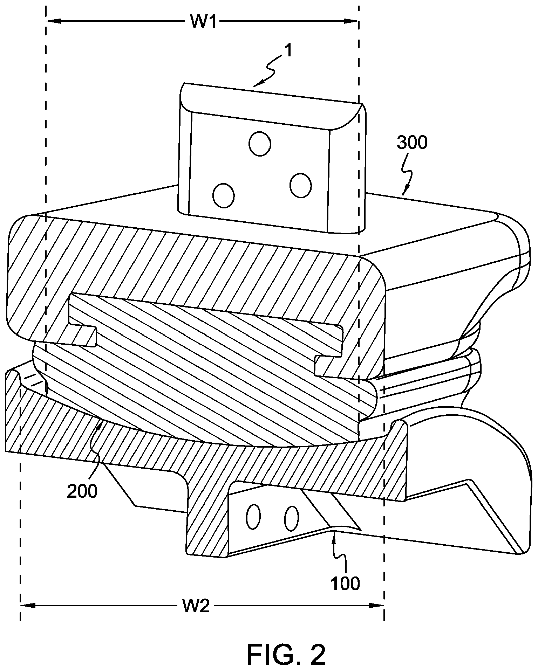

As shown in Figures 1 and 2, an ankle prosthesis implant 1 is disclosed. In one embodiment, the ankle prosthesis implant 1 includes three main components: a talar implant 100, a bearing component 200, and a tibial implant 300. Each of these components is described in further detail herein.

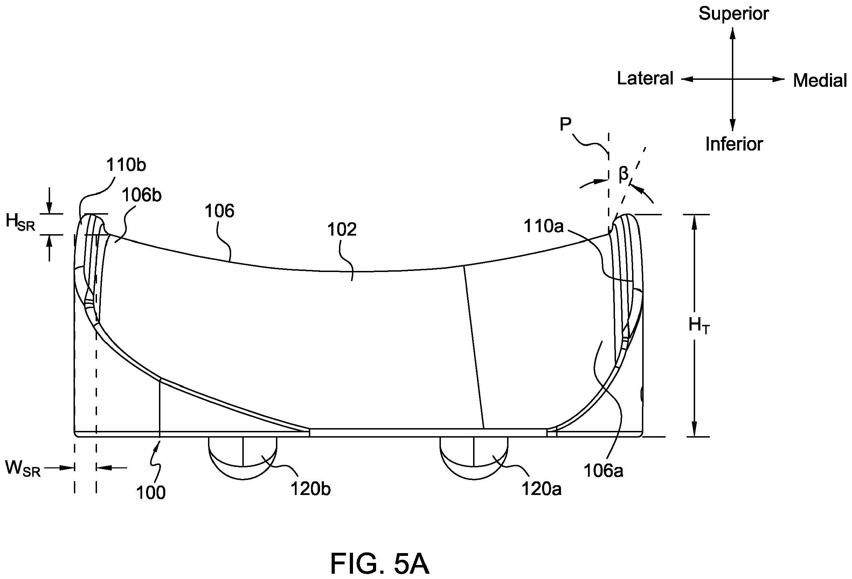

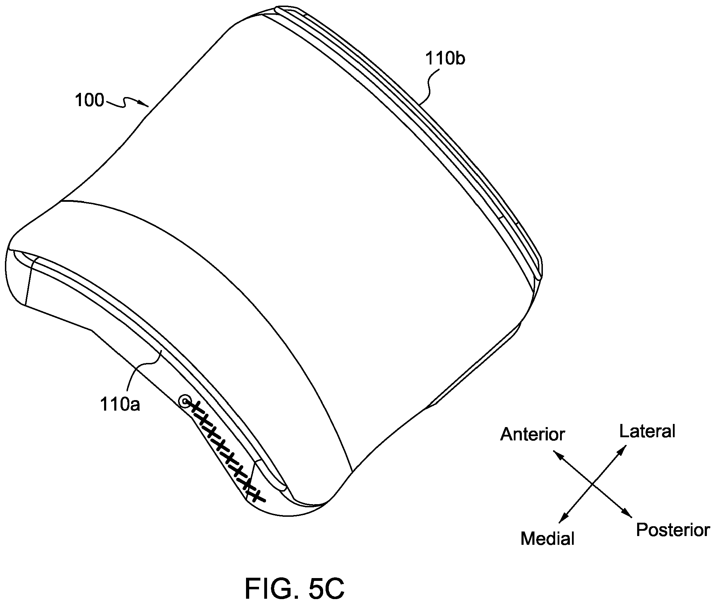

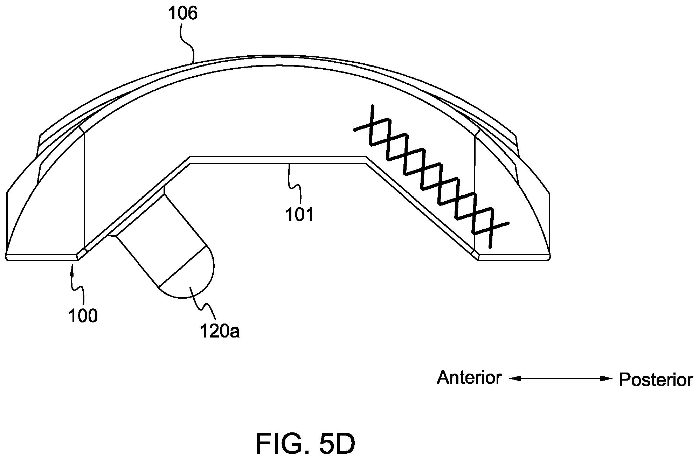

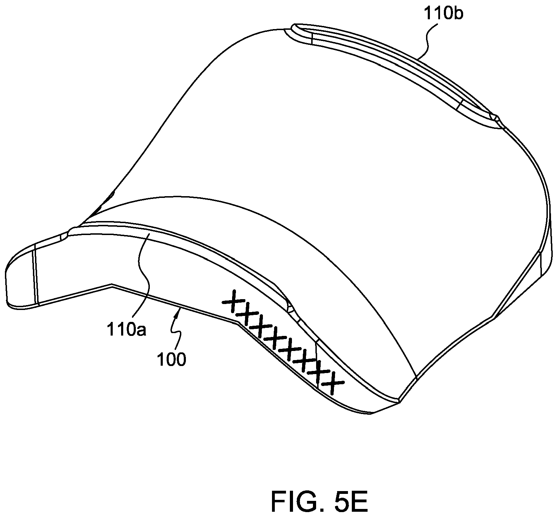

The talar implant 100 defines an inferior bone contacting region 101 (shown in Figure 5D), and a superior bearing surface 102. The inferior bone contacting region 101 is generally configured to provide a contact surface with a bone in a patient's foot. The inferior bone contacting region 101 includes at least one bone attachment protrusion 120a, 120b, as shown in Figure 5A. The at least one bone attachment protrusion 120a, 120b is generally dimensioned to extend inside of patient's bone.

The superior bearing surface 102 includes a convex portion or curvature 104 and a concave portion or curvature 106. The convex portion 104 and the concave portion 106 are both defined in various regions of the bearing surface 102, depending on which direction and through which plane that the bearing surface 102 is viewed from.

In one aspect, the superior bearing surface 102 has a hyperbolic paraboloid profile, and more specifically has a truncated hyperbolic paraboloid profile. The surface 102 is formed as doubly ruled surface. In other words, the profile includes two sets of mutually skewed lines to form the surface 102, and forms a "saddle surface." More details of the surface 102 are provided herein.

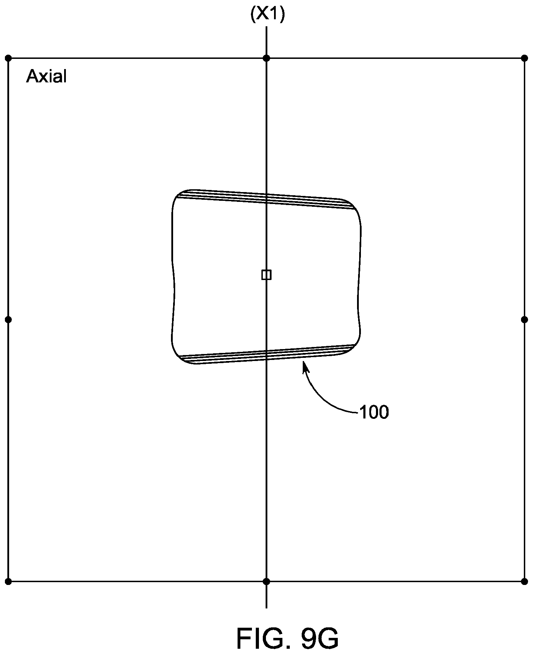

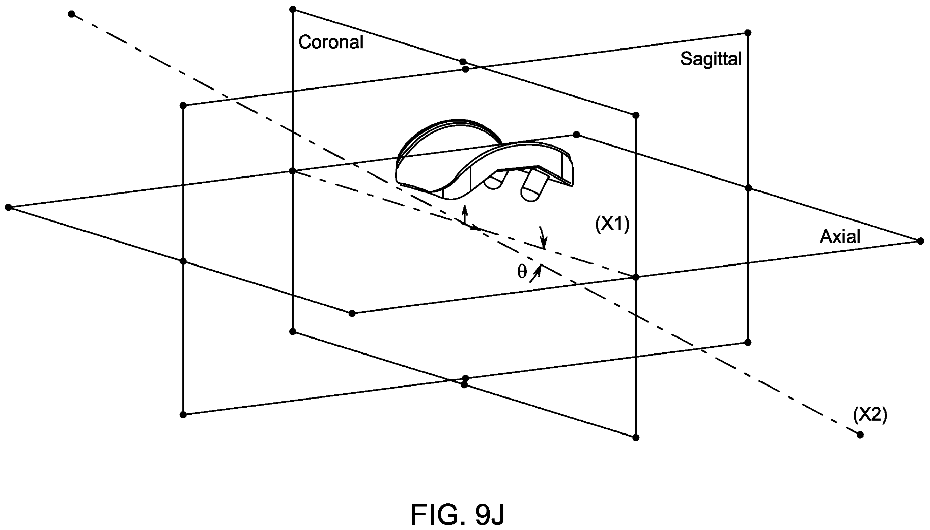

The convex portion 104 is defined in an anterior-posterior direction when viewed from the sagittal plane and has a neutral axis (X1) defined in the coronal plane at approximately the anterior-posterior midline of the talar implant and extending in the medial-lateral direction. As explained above, the term approximately means the middle 50% (+/-5%) of the anterior-posterior length of the talar implant. The positioning of the neutral axis (X1) is best shown in Figure 9G. The term anterior-posterior midline is used to generally refer to a middle region of the talar implant in the anterior-posterior direction. As used herein, the term neutral axis is defined as an axis that extends perpendicular to the sagittal plane and contains the center point of the convex curvature of the talar implant when viewed in the sagittal plane at the midline of the medial-lateral width of the talar implant. The midline of the medial-lateral width is further defined as the midpoint of the width when viewed in a coronal plane cross section taken through the most inferior point on the talar implant.

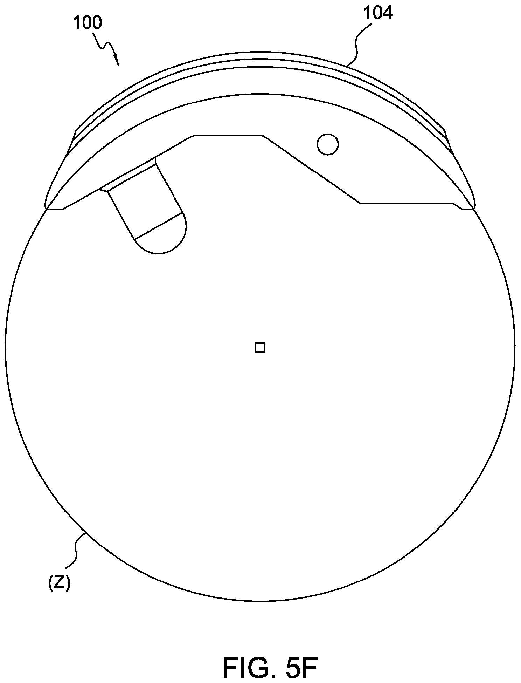

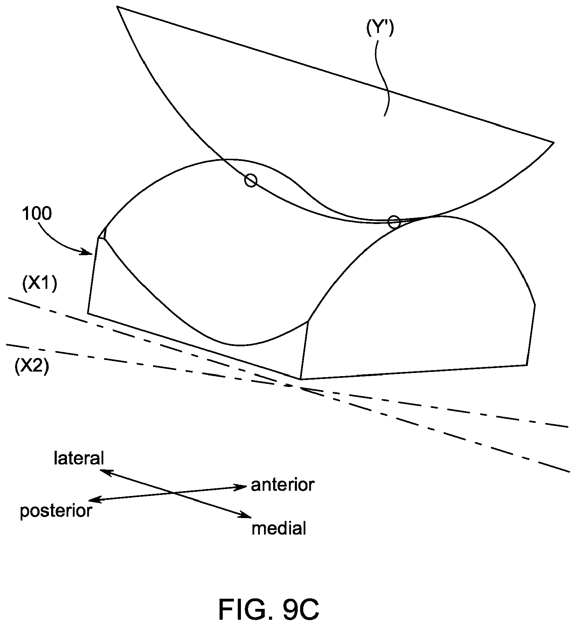

In one embodiment, the convex portion 104 can consist entirely of a single convex profile in any given sagittal plane. The convex portion 104 is illustrated with a single radius of curvature (Z) when viewed from the sagittal plane, as shown in Figures 5F and 9C. One of ordinary skill in the art would understand that the profile of the convex portion 104 can include a single radius or multiple radii in any given sagittal plane.

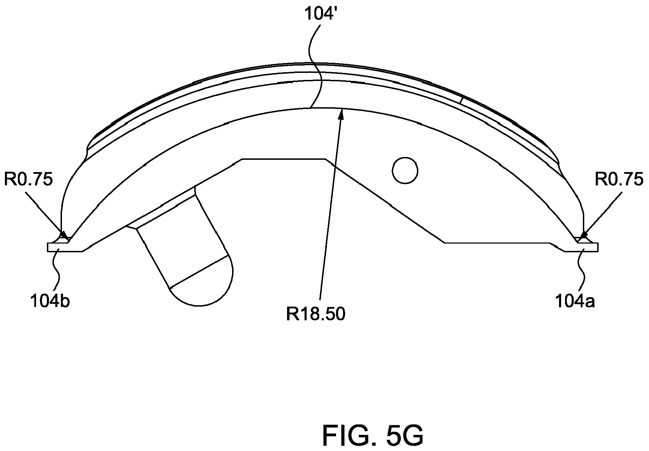

As shown in Figure 5G, the convex portion 104' can have multiple radii of curvature. In one embodiment, the convex portion 104' further includes a posterior end portion 104a and an anterior end portion 104b that each transition from a convex profile to a concave profile, as shown in Figure 5G. The posterior end portion 104a and anterior end portion 104b can also include flat regions having no curvature. Although the posterior end portion 104a and anterior end portion 104b are illustrated as having a similar curvature and flat portion to each other in Figure 5G, one of ordinary skill in the art would understand that the profiles do not have to be identical or similar.

The concave portion 106 is defined in the medial-lateral direction when viewed from the coronal plane. The concave portion 106 is swept about a secondary axis (X2) that is angled relative to the neutral axis (X1) in the medial-lateral direction by an angle (θ). In other words, the secondary axis (X2) is angled relative to the sagittal plane and the axial plane, and the angle of the secondary axis (X2) effectively sweeps the concave portion 106 to form a saddle profile for the superior bearing surface 102. The secondary axis (X2) is the primary axis upon which the bearing component can articulate about the talar component. The concave portion 106 is formed by rotating the concave portion 106 about the secondary axis (X2). One of ordinary skill in the art would understand that the concave portion 106 could be formed in a variety of ways, *e.g.* 3-D printing. When the bearing component 200 articulates on the talar implant 100 it follows the secondary axis (X2). However, because the bearing component 200 is free to slide in the medial and lateral directions, there is not a single axis of rotation of the bearing component 200 relative to the talar implant 100.

As shown in Figure 2, an interfacing surface of the bearing component 200 has a width (W1) in the medial-lateral direction that is less than a width (W2) of an interfacing surface of the talar implant 100 in the medial-lateral direction. The width (W2) of the talar implant 100 allows the bearing component 200 to articulate by at least 1° in inversion and eversion, and in another embodiment allows at least 2° in inversion and eversion.

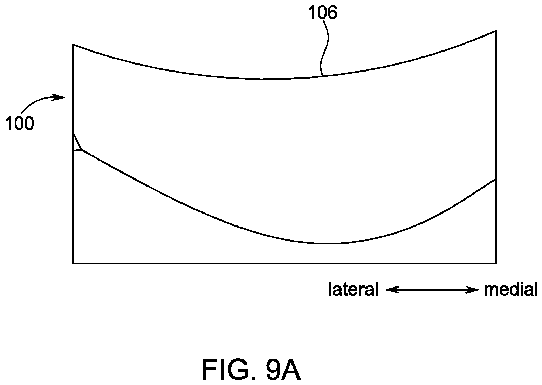

Figures 9A-9L illustrate other features of the concave portion 106 of the superior bearing surface 102 in more detail. In one embodiment, the angle (θ) of the secondary axis (X2) relative to the neutral axis (X1) is between 1° to 30° or -1° to -30°. In another embodiment, the angle (θ) of the secondary axis (X2) relative to the neutral axis (X1) is between 1° to 15°, and is angled upwards towards a medial side of the talar implant 100. In another embodiment, the angle (θ) of the secondary axis (X2) relative to the neutral axis (X1) is between 5° to 10°, and is angled upwards towards the medial side of the talar implant 100. In a more preferred embodiment, the angle (θ) of the secondary axis (X2) relative to the neutral axis (X1) is 7°, and is angled upwards towards the medial side of the talar implant 100.

This angle (θ) provides a sweeping profile of the concave portion 106, and allows for internal rotation of the talar implant 100 with plantar flexion. The specific values of the angle (θ) were selected as providing improved range of motion. Specifically, this angle (θ) gives coupled plantar flexion with internal rotation of the talar implant 100 and dorsal flexion with external rotation of the talar implant 100. This range of motion in multiple directions is critical for walking and mobility in a patient after the ankle prosthesis implant 1 is implanted.

The ankle prosthesis implant 1 provides independent inversion and eversion through the range of motion, as well as in the dorsiflexed, plantarflexed, and neutral foot. This is a result of the concave saddle shape of the talar implant being continuous from the medial to lateral direction. Existing implants prevent medial-lateral motion. The embodiments disclosed herein prevent the medial and lateral motion at the edges via the siderails, or simply as a result of the concavity. The ankle prosthesis implant 1 provides the approximate flexion angle range when heel striking occurs during a person's gait, as well as absorption of a person's foot impacting the ground during a wide range of required motion, such as smaller steps or shuffling, pivoting, uneven terrain environments, *etc.*

The saddle shape of the talar implant 100 generally provides a specific amount independent range of motion, for inversion and eversion, that is not coupled with flexion-extension or internal-external rotation. The saddle shape of the talar implant 100 reduces the forces and stresses on both the bone-implant interface and stabilizing soft tissues, by providing an extra degree of freedom.

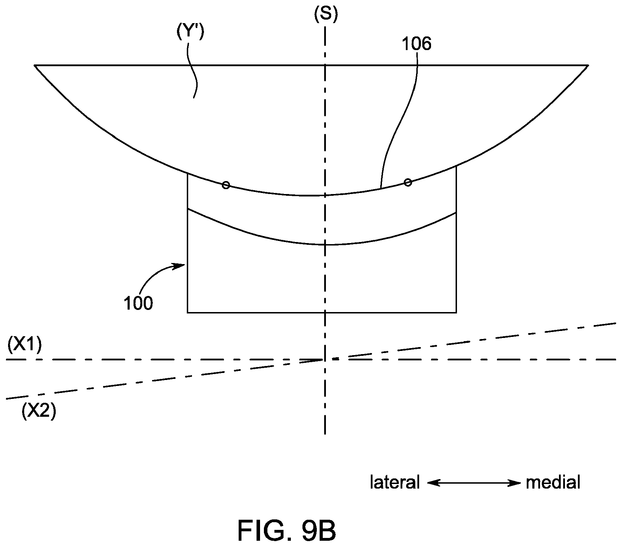

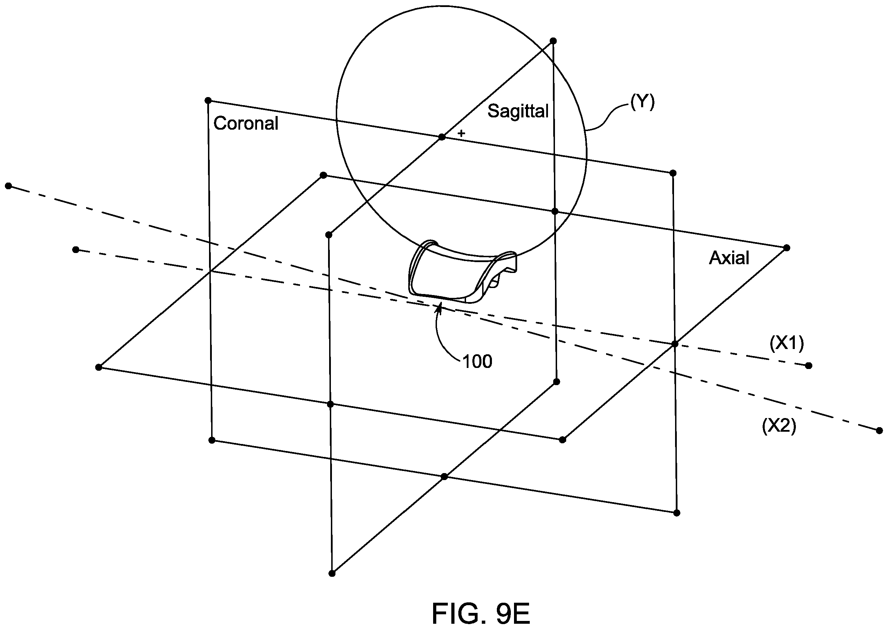

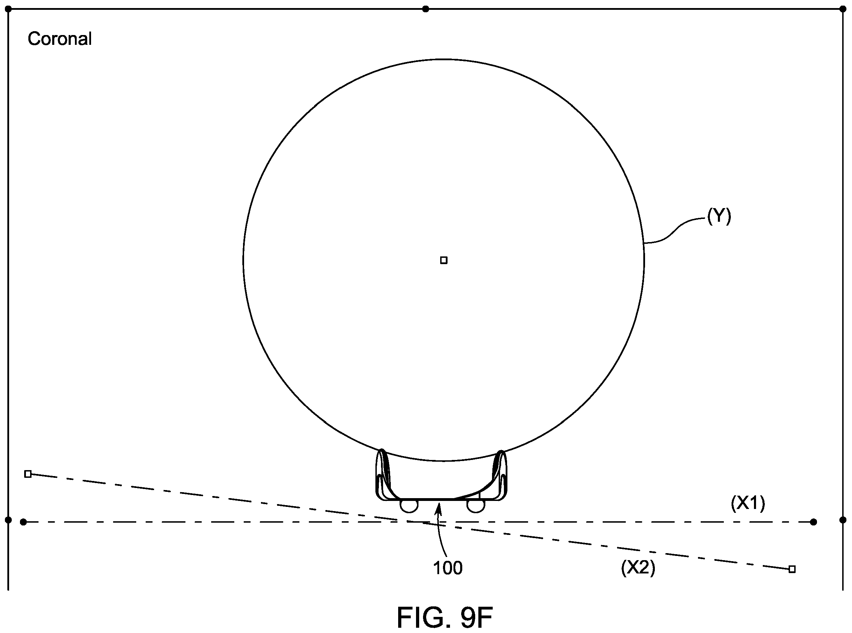

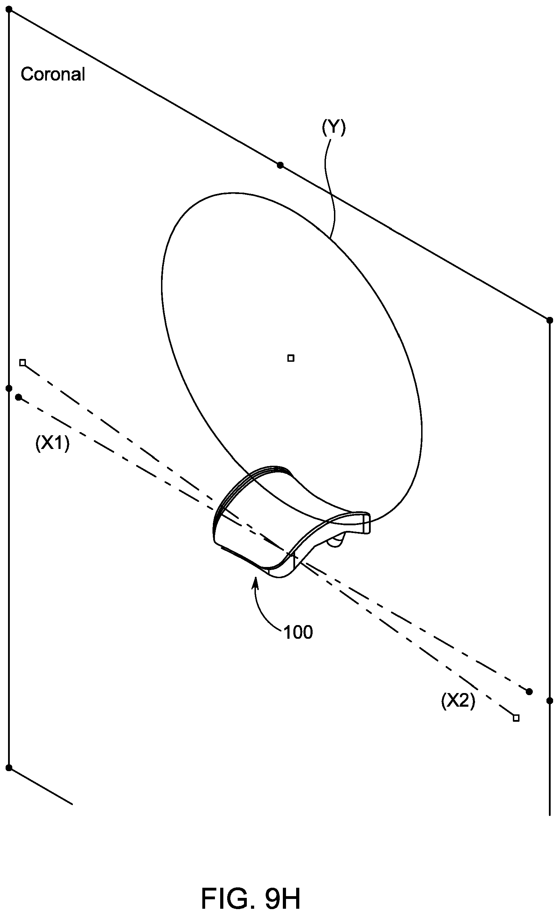

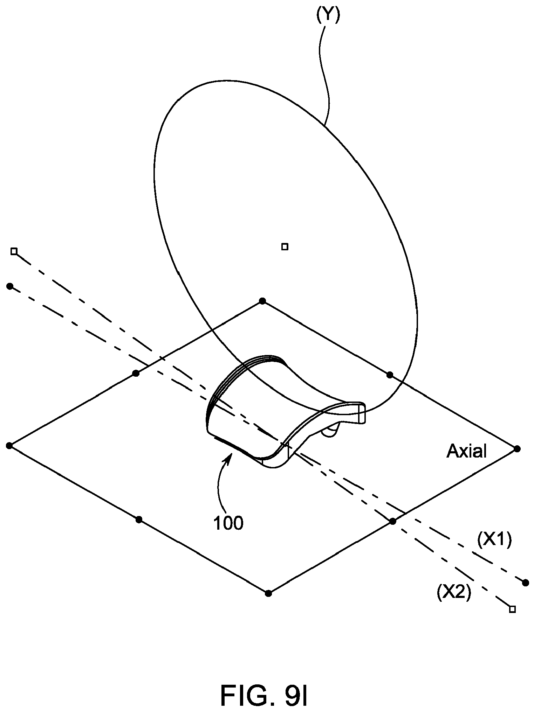

Additional features of the concave portion 106 are described herein. In one embodiment, as shown in Figure 9F, the concave portion 106 can have a single radius of curvature when viewed from the coronal plane. The radius of curvature is defined by the reference circle (Y) in Figures 9E, 9F, 9H and 9I. A portion of the reference circle (Y') is also illustrated in Figures 9B and 9C. The concave portion 106 can also include multiple radii of curvature, such as a radius of curvature in a medial region, central region, and lateral region.

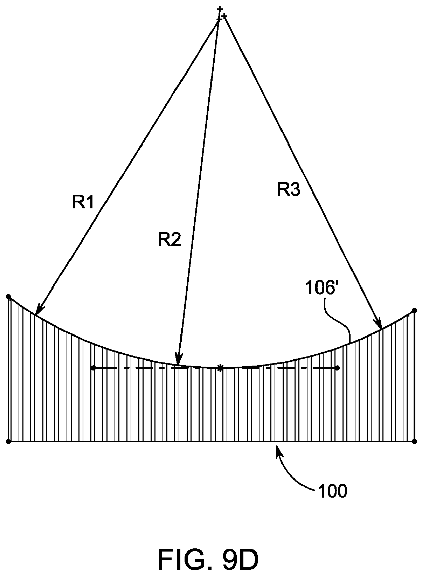

In other embodiments, such as shown in Figure 9D, the concave portion 106' can have at least two radii of curvature when viewed in the coronal plane. Three radii of curvature R1, R2, R3 are illustrated in Figure 9D. One of ordinary skill in the art would understand based on the present disclosure that any number of radii can be selected to provide the desired bearing surface of the concave portion 106. Additionally, R1 and R3 can be equal to each other, and R1 and R3 can be greater than or less than R2. Any relationship between these radii can be selected depending on the desired overall geometry of the concave portion 106. Generally, the radii R1 and R3 are between 1-25% of the radius R2, or between 75-125% of R2. In one embodiment, the radii R1 and R3 are approximately 99% of the Radius R2. In one embodiment, the radii R1 and R3 are approximately 2% of R2.

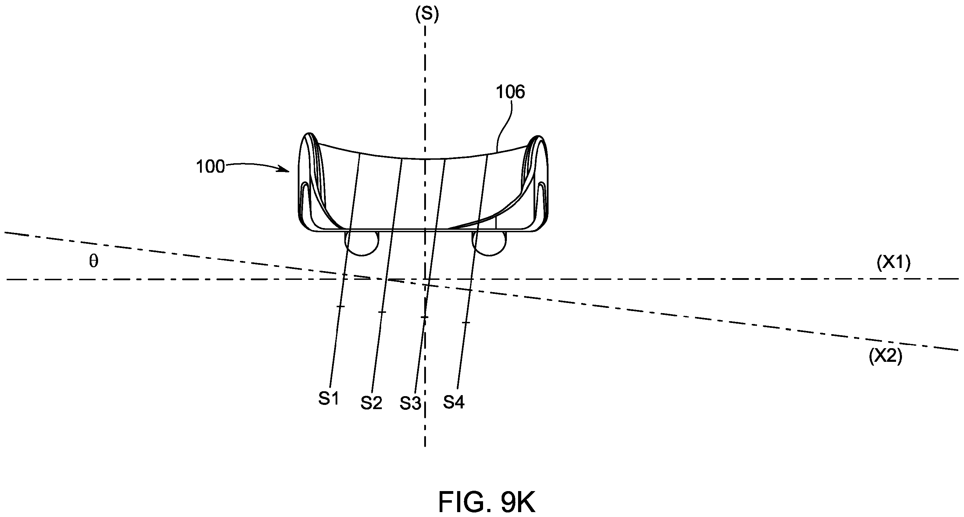

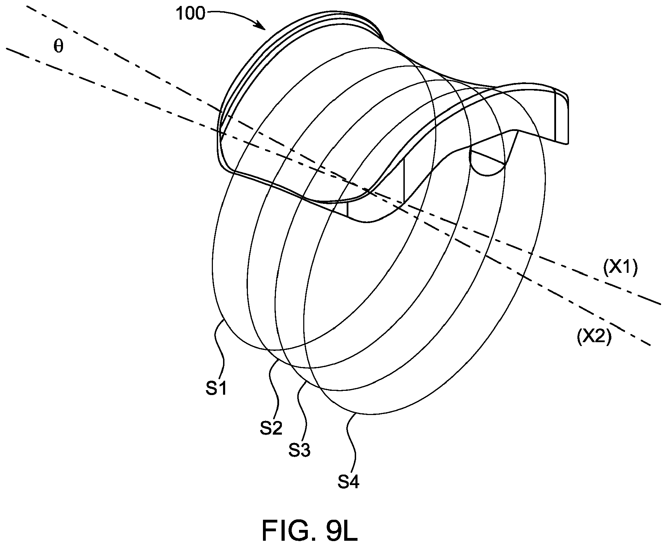

Figures 9K and 9L provide further definition for the representative circles, *i.e.* sweeping curves. Four representative cross-sectional circles S1, S2, S3, S4 are illustrated in Figures 9K and 9L. These circles are driven by, or are a resultant of, the concave portion 106 and its axis of rotation about the secondary axis (X2). Each of the cross-sectional circles S1, S2, S3, S4 are positioned along the secondary axis (X2) and extend perpendicular or normal to the secondary axis (X2). The cross-sectional circles S1, S2, S3, S4 are angled relative to the neutral axis (X1) and the sagittal plane (S). Although only four cross-sectional circles S1, S2, S3, S4 are illustrated, one of ordinary skill in the art would understand that the profile of the concave portion 106 can be composed of any number of these circles. Cross-sectional circle S1 is defined on the medial side of the talar implant 100, and cross-sectional circle S4 is defined on the lateral side of the talar implant 100. Based on the sloped concave portion 106, cross-sectional circle S1 is above or raised compared to cross-sectional circle S4. Although the cross-sectional circles S1, S2, S3, S4 are only illustrated in some of the drawings, one of ordinary skill in the art would understand that this profile is present in all other embodiments of the implant.

The width of the bearing component 200 is less than the width of the talar implant 100. This allows distinct inversion-eversion motion of the bearing component 200 relative to the talar implant 100, while still maintaining substantial contact between the articulating surfaces. In other words, the bearing component 200 can rotate or translate up the side surfaces formed by the saddle profile of the talar implant 100. The length (L) of the talar implant influences flexion-extension range of motion, but not varus-valgus. Varus-valgus (also described as inversion-eversion) is dictated by the width (Ws) of the talar implant, the width of the articulating surface of the bearing component, and the radii of curvature of the concave surface on the talar implant.

In one embodiment, the width (W_{S}) of the concave portion 106 in the medial-lateral direction when viewed from an axial plane is less than an overall width (W_{O}) of the talar implant 100 in the medial-lateral direction when viewed from the axial plane. The overall width (W_{O}) of the talar implant 100 is defined between an outermost medial edge 105a and an outermost lateral edge 105b. In one embodiment, the width (W_{S}) is between 80% - 99% of the overall width (W_{O}).

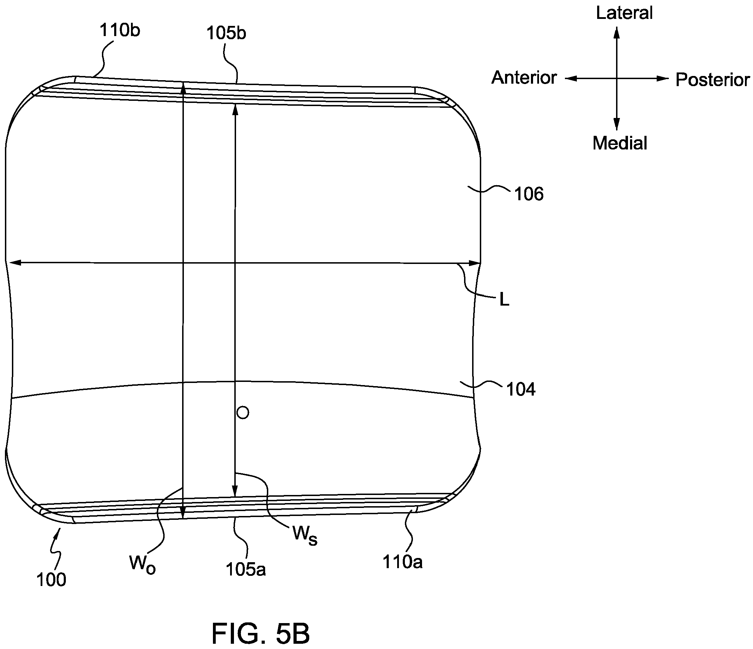

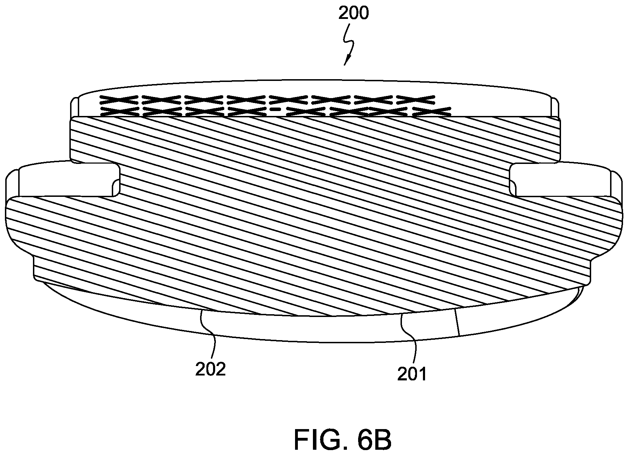

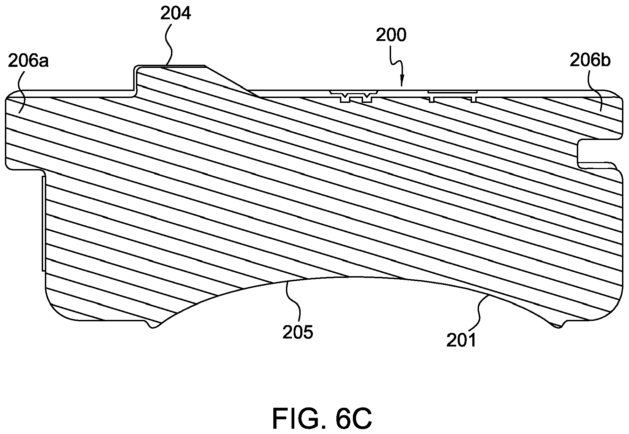

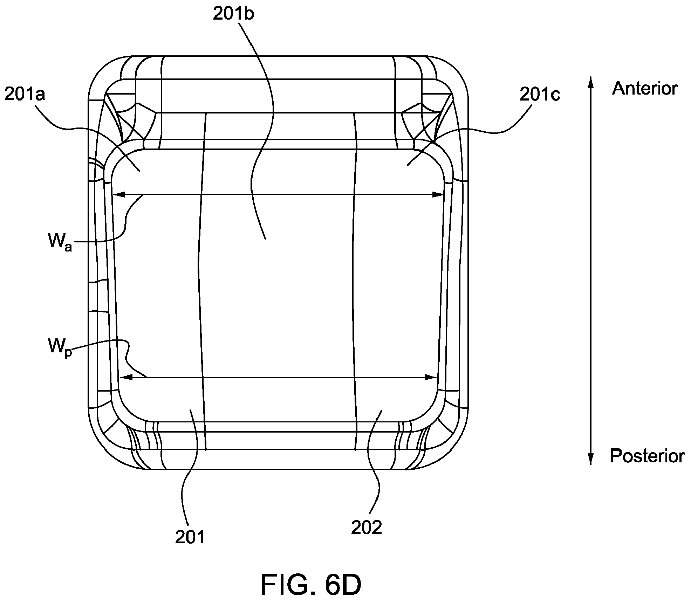

As shown in Figure 5B, the width (W_{S}) of the concave portion 106 and the width (W_{O}) of the talar implant 100 both taper inward along the anterior-posterior direction. Similarly, the bearing component 200, which engages these surfaces of the talar implant 100, can also include a mating surface 201 that tapers in a complementary manner as the width (W_{S}) of the concave portion 106. The tapering on the corresponding surfaces of the bearing component 200 is best shown in Figure 6D, whereas an anterior width (Wₐ) of the bearing component 200 is greater than a posterior width (Wₚ). In one embodiment, a width of the mating surface 201 is less than the width (W_{S}) of the concave portion 106, which provides for relative inversion and eversion, or medial-lateral displacement.

One of ordinary skill in the art would understand that the respective surfaces on the talar implant 100 and the bearing component 200 may not include tapered profiles.

As best shown in Figure 5A, a lateral end 106a and a medial end 106b of the concave portion 106 both transition to a respective siderail 110a, 110b which partially define the outermost medial edge 105a and the outermost lateral edge 105b of the talar implant 100. In other words, the concave portion 106 does not extend for an entire medial-lateral extent of the talar implant 100. As shown in Figure 5A, the siderails 110a, 110b are angled by a siderail angle (β) from a vertical plane (P) extending in a superior-inferior direction when viewed from the coronal plane. In one embodiment, the siderail angle (β) is between -30° to 60°. Based on this configuration, a 0° siderail is a vertical wall. A negative value for siderail angle represents a wall angled toward the midline of the implant and may provide greater stability than a wall with a positive siderail angle which is angled toward the exterior of the implant. The siderails 110a, 110b prevent excessive translation and/or inversion and eversion. As shown in Figure 5B, the siderail 110b on the lateral side tapers inward when going from the anterior to posterior direction. The siderail 110a on the medial side, as shown in Figure 5B, does not taper as much or at all, compared to the siderail 110b.

As shown in Figure 5A, the siderails 110a, 110b each have a siderail height (H_{SR}) in a superior-inferior direction when viewed in the coronal plane that is at least 0 mm and less than 6.0 mm. In one embodiment, the siderail height (H_{SR}) is at least 1%-15% of a total height (H_{T}) of the talar implant 100 in the superior-inferior direction when viewed in the coronal plane.

In one embodiment the siderails are omitted and do not exist. The amount of constraint, or limitation on the range of motion or translation in the medial lateral direction is a function of the siderail in addition to the concave surface. The addition of a siderail provides additional constraint to the implant construct limiting excessive motion that may be present when normal range of motion is exceeded (i.e. walking on uneven ground, spraining or "rolling the ankle", etc.).

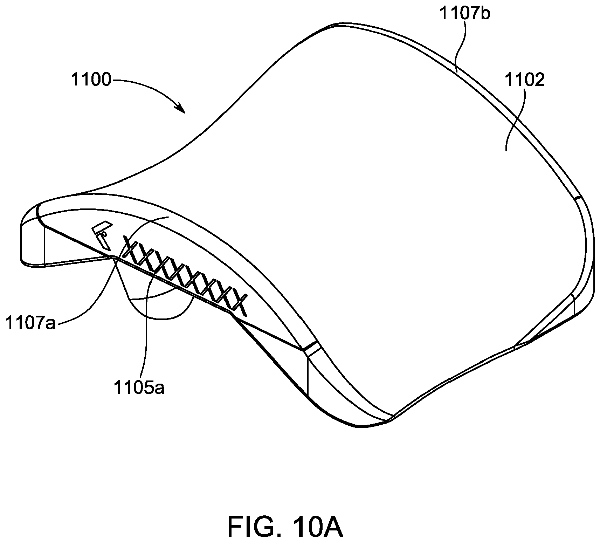

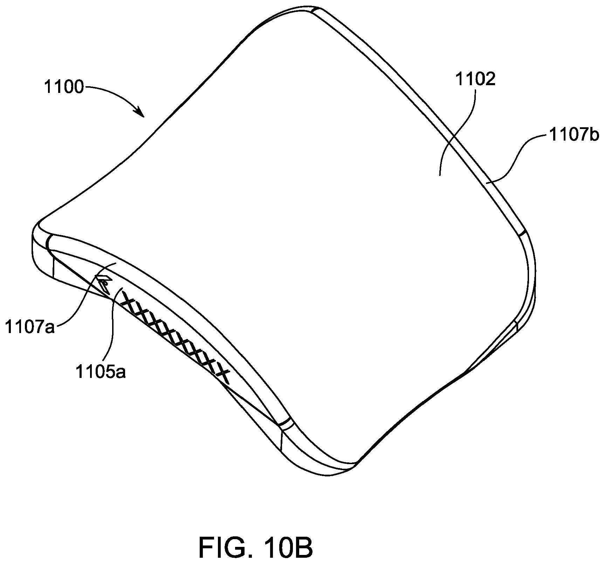

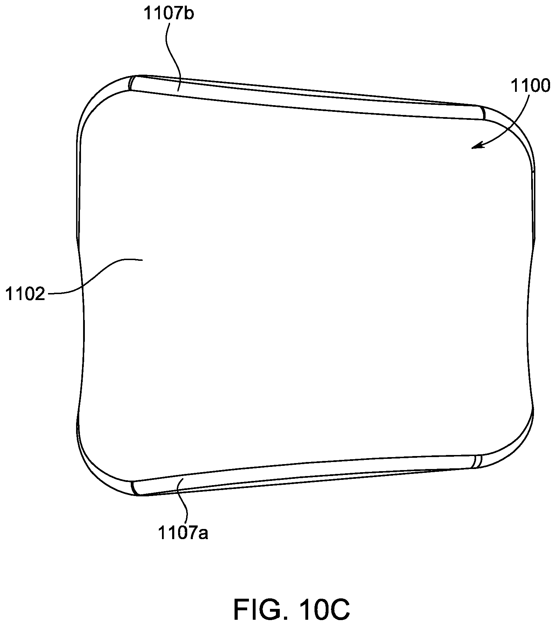

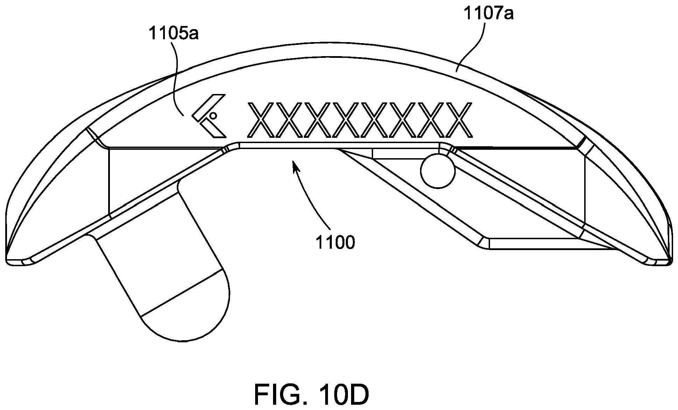

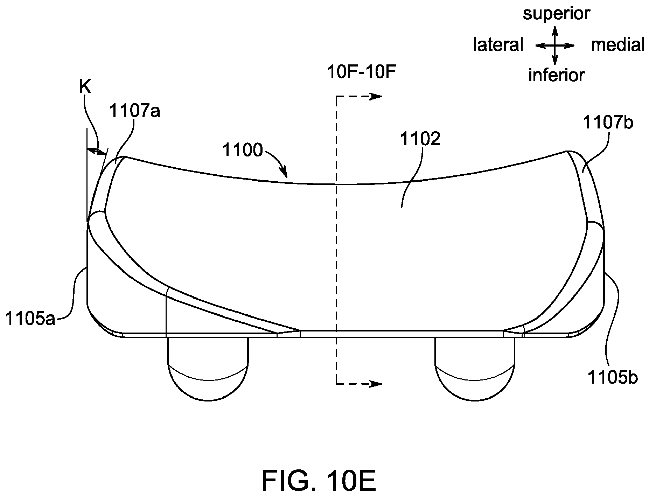

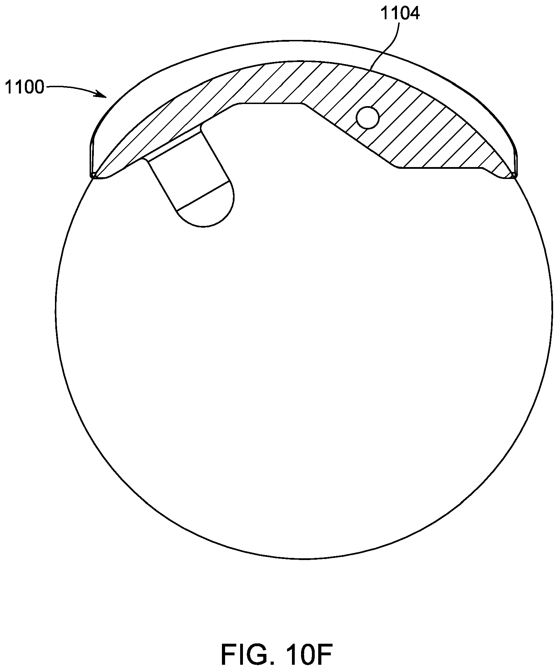

Figures 10A-10F illustrate an embodiment of a talar implant 1100 that lacks siderails. The talar implant 1100 is otherwise identical to the talar implant 100 described herein unless features are otherwise described and distinctions are specified. As best shown in Figure 10E, the talar implant 1100 includes sidewalls 1105a, 1105b defined on the lateral and medial terminal edges of the talar implant 1100. Between these sidewalls 1105a, 1105b and a superior bearing surface 1102, the talar implant 1100 includes fillets 1107a, 1107b which define transitional areas between the superior bearing surface 1102 and the sidewalls 1105a, 1105b. In contrast to the siderails 110a, 110b of other embodiments and configurations, the fillets 1107a, 1107b define a relatively smoother transition between the superior bearing surface 1102 and the sidewalls 1105a, 1105b, such that the curved profile of the superior bearing surface 1102 is continuous to the sidewalls 1105a, 1105b. The talar implant 1110 is configured to be used with the bearing component 200 and the tibial implant 300.

In one embodiment, as shown in Figure 10E, the sidewalls 1105a, 1105b are tapered in the medial lateral direction for at least a portion of the length of the implant. An angle (K) of the sidewall taper in the medial lateral direction may be between -60° and +60°, wherein a positive angle defines a taper toward the medial-lateral midline of the talar component and a negative angle defines a taper away from the medial-lateral midline of the talar component. In one embodiment, an angle (K) of the sidewall taper is between -45° and +45°. In another embodiment, the angle (K) of the sidewall taper is between -30° and +30°.

Although not explicitly annotated, the angle of the sidewall taper can also be the same in the other embodiments.

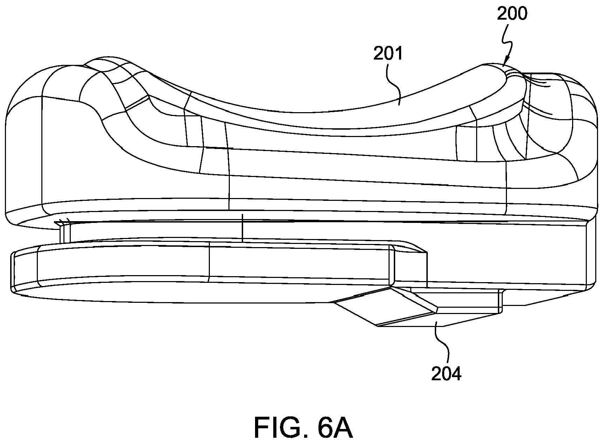

The bearing component 200 articulates with at least the talar implant 100 and also possibly with the tibial implant 300. The bearing component 200 defines a mating surface 201 that abuts the superior bearing surface 102 and articulates with the talar implant 100. As shown in Figures 6A-6E, the mating surface 201 of the bearing component 200 includes a concave bearing surface 205 when viewed in the sagittal plane and a convex bearing surface 202 when viewed in the coronal plane.

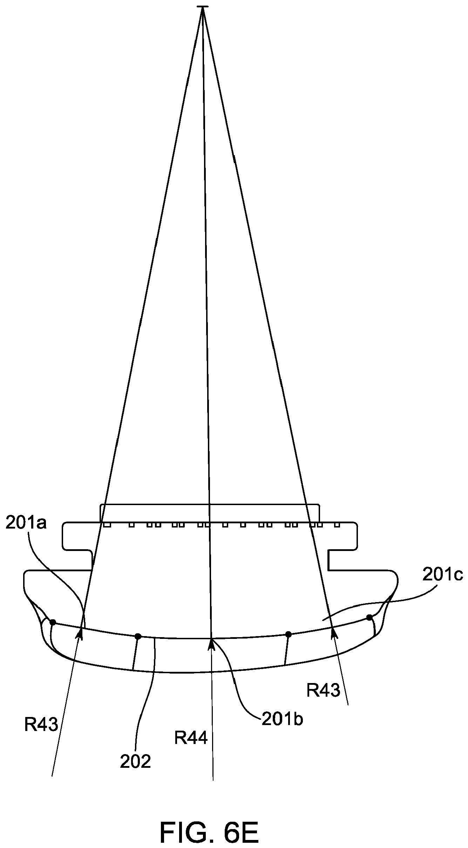

Referring to Figures 6D and 6E, in one embodiment at least a portion (*i.e.* portion 201b) of the convex bearing surface 202 of the bearing component 200 is congruent with a substantial portion of the concave portion 106 of the talar implant 100. As used in this instance, the term substantial means the interfacing surfaces are congruent for at least 50% (*i.e.* a majority) of a surface area of the respective bearing surfaces. The congruent portion 201b is illustrated as middle section of the convex bearing surface 202. In one embodiment, these components are not congruent. For example, the bearing component 200 can have a relatively smaller or larger convex radius than the corresponding concave portion 106 of the talar implant 100. In another embodiment, the bearing component 200 has a convex radius that is 50% of the concave radius of the corresponding concave portion 106 of the talar implant 100.

In another embodiment, outer portions of the convex bearing surface 202 of the bearing component 200 (*i.e.* end surfaces 201a, 201c) are offset from the concave portion 106 of the talar implant 100 in a variable manner. In other words, the end surfaces 201a, 201c are not complementary or congruent to the concave portion 106 of the talar implant 100. The offset portions 201a, 201c are illustrated as outer sections of the convex bearing surface 202.

The bearing component 200 further includes a bearing lock surface 204, and support regions 206a, 206b that are adapted and dimensioned to interface with the tibial implant 300. The combination of the bearing lock surface 204 and support regions 206a, 206b allows the bearing component 200 to be slid into engagement with correspondingly shaped regions of the tibial implant 300, which are described in more detail herein. In another embodiment, the bearing component is designed to articulate with the tibial component and does not include a lock surface or additional support regions.

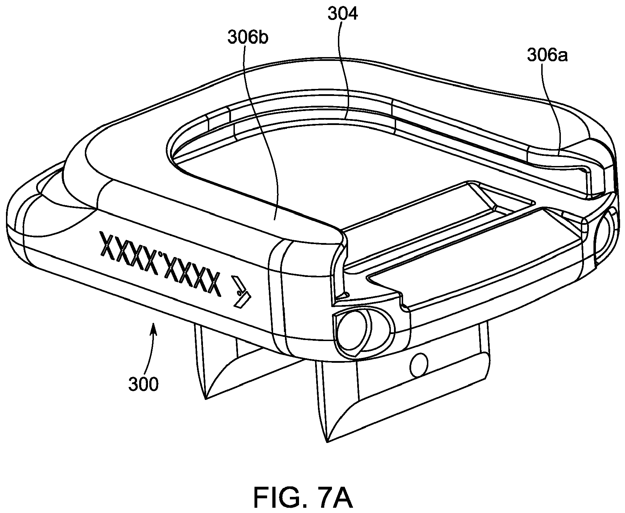

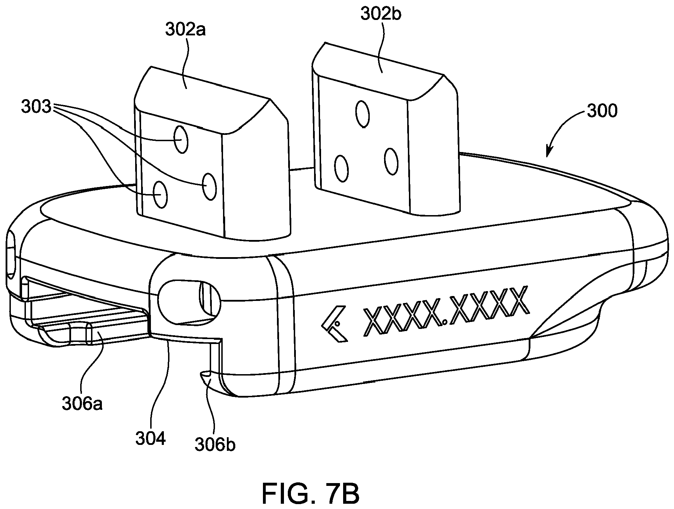

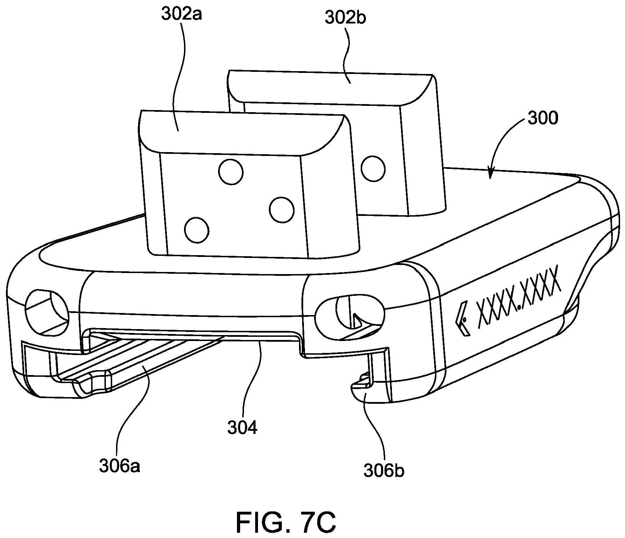

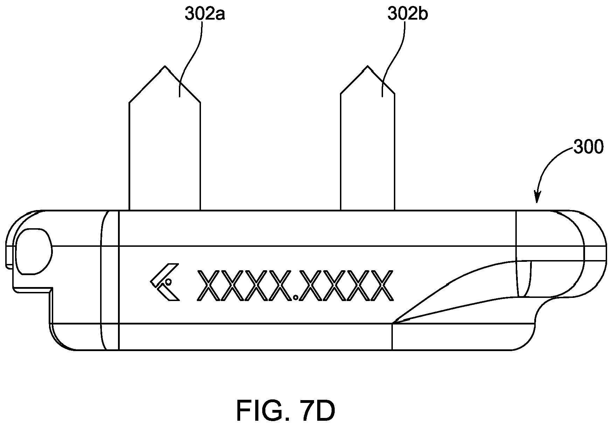

The tibial implant 300 is more clearly shown in Figures 7A-7D. The tibial implant 300 includes at least one dorsal fin 302a, 302b. Although two fins 302a, 302b are illustrated in the drawings, one of ordinary skill in the art would understand that one or more than two fins can be used. Additionally, the term fin is used herein to broadly refer to any raised element, and does not limit the specific shape on these elements. The at least one dorsal fin 302a, 302b extends in the medial-lateral direction and extends perpendicular from a superior planar surface or upper surface of the tibial implant 300. The term perpendicular, as used in this instance, means that the fins 302a, 302b extend generally upward from a planar surface. The fins 302a, 302b may, but are not required, extend exactly 90° from the planar surface.

The at least one dorsal fin 302a, 302b further includes at least one of a void, opening, or hole 303. Although three voids, openings, or holes 303 are illustrated in the drawings, one of ordinary skill in the art would understand based on the present disclosure that any number of voids, openings, or holes 303 can be provided. These voids, openings, or holes 303 are generally provided to promote adhesion or attachment of the tibial implant 300 with a patient's bone.

The tibial implant 300 further includes a channel 304 defined on a lower or inferior surface. The channel 304 is defined by at least two siderails 306a, 306b that are dimensioned to receive a portion of the bearing component 200. The channel 304 is dimensioned to receive a portion of the bearing component 200, and more specifically receives the support regions 206a, 206b of the bearing component 200. A lock slot 308 is defined on the inferior surface of the tibial implant 300 and is dimensioned to receive the bearing lock surface 204. Although specific shapes, sizes, and geometries are illustrated for the mating features of the bearing component 200 (*i.e.* the bearing lock surface, support regions 206a, 206b, *etc.*) and the tibial implant 300 (*i.e.* the channel 304, siderails 306a, 306b, lock slot 308, *etc.*)*,* one of ordinary skill in the art would understand based on the present disclosure that these components may be modified. Each of these corresponding features on the bearing component 200 and the tibial implant 300 are generally shaped to be complementary to each other.

Although a single talar implant 100 is shown and described herein, one of ordinary skill in the art would understand from this disclosure that a similar talar implant 100 would be provided for a patient's opposite ankle. The talar implant for an opposite ankle would include identical features, but oriented to conform to the patient's opposite ankle. One of ordinary skill in the art would also recognize from this disclosure that the size of the talar implant can vary, depending on the size of the patient in which the talar implant is being used.

Additionally, the talar implant 100 can be used independently of any tibial implant 300.

The embodiments disclosed herein generally provide flexion and extension of the ankle joint (when viewed in the sagittal plane), along with internal/external rotation (*i.e.* rotation about a vertical axis of a patient's foot) that is coupled with the flexion/extension and along with independent inversion and eversion. The embodiments disclosed herein generally provide at least 3° of total rotation coupled with flexion and 3° of rotation coupled with extension.

Having thus described the present invention in detail, it is to be appreciated and will be apparent to those skilled in the art that many physical changes, only a few of which are exemplified in the detailed description of the invention, could be made without altering the inventive concepts and principles embodied therein.

It is also to be appreciated that numerous embodiments incorporating only part of the preferred embodiment are possible which do not alter, with respect to those parts, the inventive concepts and principles embodied therein.

The present embodiment and optional configurations are therefore to be considered in all respects as exemplary and/or illustrative and not restrictive, the scope of the invention being indicated by the appended claims rather than by the foregoing description, and all alternate embodiments and changes to this embodiment which come within the meaning and range of equivalency of said claims are therefore to be embraced therein.

## Claims

1. An ankle prosthesis implant comprising:
a talar implant defining a superior bearing surface, the superior bearing surface including:
a convex portion defined in an anterior-posterior direction when viewed from a sagittal plane and having a neutral axis (X1) defined in a coronal plane at an anterior-posterior midline of the talar implant and extending in a medial-lateral direction, and
a concave portion defined in the medial-lateral direction when viewed from the coronal plane, the concave portion being swept about a secondary axis (X2) that is angled relative to the neutral axis (X1) in the medial-lateral direction upwards towards a medial end of the talar implant by an angle (θ).

2. The ankle prosthesis implant of claim 1, wherein the angle (θ) of the secondary axis (X2) relative to the neutral axis (X1) is tilted upwards by 1° to 30°, particularly 5° to 10°, and more particularly 7°, toward the medial end of the talar implant.

3. The ankle prosthesis implant of claim 1, wherein the concave portion has at least one radius of curvature when viewed from the coronal plane, and the at least one radius of curvature is always concave.

4. The ankle prosthesis implant of claim 1, wherein a width (W_{S}) of the concave portion in the medial-lateral direction when viewed from an axial plane is less than an overall width (W_{O}) of the talar implant in the medial-lateral direction when viewed from the axial plane, the overall width (W_{O}) of the talar implant being defined between an outermost medial edge and an outermost lateral edge.

5. The ankle prosthesis implant of claim 1, wherein a lateral end and a medial end of the concave portion both transition to a respective siderail which partially defines an outermost medial edge and an outermost lateral edge of the talar implant.

6. The ankle prosthesis implant of claim 1, wherein the talar implant defines an inferior bone contacting region that includes at least one bone attachment protrusion which is dimensioned to extend inside of a bone.

7. The ankle prosthesis implant of claim 1, wherein the convex portion is comprised of a plurality of curves including a transitional region defined in at least one anterior end of the talar implant, and the transitional region includes at least one of a concave profile or a flat profile.

8. The ankle prosthesis implant of claim 1, further comprising a bearing component defining a mating surface that abuts the superior bearing surface and articulates with the talar implant, the mating surface including a concave bearing surface when viewed in the sagittal plane and a convex bearing surface when viewed in the coronal plane.

9. The ankle prosthesis implant of claim 8, wherein at least a portion of the convex bearing surface of the bearing component is congruent with a substantial portion of the concave portion of the talar implant, outer portions of the convex bearing surface of the bearing component are offset from the concave portion of the talar implant, and a medial to lateral width of the bearing component is less than a medial to lateral width of the talar implant.

10. The ankle prosthesis implant of claim 1, further comprising a tibial implant including at least one dorsal fin that extends in the medial-lateral direction and extends perpendicular from a superior planar surface of the tibial implant, and the at least one dorsal fin includes at least one of a void, a opening, or a hole.

11. The ankle prosthesis implant of claim 1, wherein the superior bearing surface has a truncated hyperbolic paraboloid profile.

12. An ankle prosthesis implant comprising:
a talar implant defining a superior bearing surface, the superior bearing surface including:
a convex portion defined in an anterior-posterior direction when viewed from a sagittal plane and having a neutral axis (X1) defined in a coronal plane at an anterior-posterior midline of the talar implant and extending in a medial-lateral direction, and
a concave portion defined in the medial-lateral direction when viewed from the coronal plane, the concave portion being swept about a secondary axis (X2) that is angled upwards relative to the neutral axis (X1) in the medial-lateral direction towards a medial end of the talar implant by an angle (θ), and the angle (θ) of the secondary axis (X2) relative to the neutral axis (X1) is tilted upwards by at least 1° toward the medial end of the talar implant; and
a bearing component that articulates with the talar implant, said bearing component defining an interfacing surface having a width (W1) in the medial-lateral direction that is less than a width (W2) in the medial-lateral direction of an interfacing surface of the talar implant, and the width (W2) of the talar implant allows the bearing component to articulate by at least 1° in inversion or eversion, and particularly wherein the bearing component is configured to articulate by at least 2° in inversion or eversion.

13. The ankle prosthesis implant of claim 1 or 12, wherein a lateral end and a medial end of the concave portion each transition to a respective fillet which (i) partially defines an outermost lateral edge and an outermost medial edge of the talar implant, and (ii) transition the concave portion to sidewalls that have a taper towards a medial region of the talar implant.

14. The ankle prosthesis implant of claim 1 or 12, wherein the convex portion has at least one radius of curvature when viewed from the sagittal plane.

15. An ankle prosthesis implant comprising:
a talar implant including a superior bearing surface having a truncated hyperbolic paraboloid profile.
